# EUROPEAN PATENT APPLICATION

(11) **EP 3 838 194 A1**
(43) Date of publication of application: **23.06.2021**
(21) Application number: 20215848.1
(22) Date of filing: 19.12.2020
(51) Int. Cl.: A61B 17/42, A61M 25/00, A61M 31/00, A61B 17/00, A61B 17/34, A61L 24/00

(54) **PERCUTANEOUSLY ADMINISTERED THERAPEUTIC SUBSTANCE FOR TREATMENT OF TARGET TISSUE**

(30) Priority: 20.12.2019 US 201962951461 P
(71) Applicant: Gyrus ACMI, Inc. d/b/a Olympus Surgical Technologies America, Southborough MA 01772 (US)
(72) Inventor: MURDESHWAR, Nikhil M., Maple Grove, Minnesota MN 55311 (US); HOLMAN, Thomas, Princeton, Minnesota MN 55371 (US)
(74) Representative: Noack, Andreas

(57) **Abstract**

A method for treating target tissue comprises introducing a portion of a surgical device into a patient, the surgical device having a treatment end, positioning the treatment end of the surgical device adjacent to the target tissue, and administering a devascularizing material to the target tissue. The devascularizing material, after a time period, is able to devascularize the target tissue.

A surgical device comprises a shaft extending between proximal and distal portions, a treatment end located at the distal portion, where the treatment end administers the devascularizating material to a target tissue.

## Description

### PRIORITY CLAIM

This application claims priority to U.S. Serial No. 62/951,461, filed on December 20, 2019, entitled "PERCUTANEOUSLY ADMINISTERED THERAPUTIC SUBSTANCE FOR TREATMENT OF TARGET TISSUE", the disclosure of which is incorporated by reference in its entirety.

### TECHNICAL FIELD

This document pertains generally, but not by way of limitation, to surgical devices and methods thereof, and more particularly, to surgical devices used for treating a target tissue such as uterine abnormalities.

### BACKGROUND

Many surgical procedures involve the treatment or removal of target tissue, e.g., diseased or unwanted tissue, located inside of a patient. As such, these procedures require access to the internal anatomy of the patient via an open procedure or through a smaller opening in minimally invasive procedures.

Women suffer from a variety of uterine abnormalities that can cause various issues. As such, in certain instances, the removal of the uterine abnormality is recommended. Endometriosis, polyps, and fibroids are examples of some uterine abnormalities that may require treatment.

Endometriosis is a complex disorder associated with pelvic pain and infertility and is characterized by the implantation of endometrial tissue outside the uterus. Endometriosis is a condition in which endometrium tissue that typically lines the inside of a uterus spreads to other places in the abdomen. The condition can be particularly painful as the endometrium tissue outside the uterus continues to behave in the manner of endometrium within the uterus during the menstrual cycle by thickening, breaking-down and bleeding. The main clinical symptoms of endometriosis are pelvic pain, bleeding and infertility.

A polyp is an abnormal growth of tissue from a mucous membrane. An endometrial or uterine polyp an abnormal growth attached to an inner wall of the uterus. Uterine polyps are usually benign, but they can be cancerous or eventually turn into cancer. Uterine fibroids are the most common pelvic tumor in women, affecting approximately one quarter of women during their reproductive years. Uterine fibroids are generally noncancerous but may potentially lead to infertility or cause adverse effects if they occur during pregnancy. Typical symptoms include abnormal bleeding, pressure, or pain.

Uterine fibroids are categorized based on location on the uterus. Sub-mucosal fibroids form on the inside wall of the uterus, sub-serosal fibroids form on the outside wall of the uterus, intra-mural fibroids form within the walls of the uterus, and pedunculated fibroids are connected to the inside or outside wall of the uterus. Typical symptoms include abnormal bleeding, pressure, or pain.

Regardless of the abnormality, the treatment of the abnormality is important to minimize pain and discomfort, maintain fertility, and prevent cancer.

### OVERVIEW

The present inventors have recognized, among other things, that problems to be solved in performing medical procedures include providing an alternative for treating various target tissues such as, but not limited to, uterine abnormalities. While the examples herein discuss uterine abnormalities, the methods and devices can be used to treat abnormalities or other target tissues located in other locations of the body such as, but not limited to, the lungs, abdomen, and colon.

Current endometriosis treatments can include pharmaceutical therapy that provides symptomatic treatment such as analgesic agents and pills and hormone therapy. Surgical treatments include tissue removal such as a hysterectomy (surgical removal of the uterus) or surgical removal of the endometrial tissue. However, the symptomatic treatments, while treating the pain, fail to treat the underlying cause and hormone therapy can have adverse side-effects for some patients. Further, surgical treatments such as a hysterectomy or cutting out a portion of tissue can cause physical and psychological trauma for patients.

Polyps can be treated by a polypectomy, which is a surgery to remove the polyp. In such instances, a hysteroscope is inserted into the uterus and surgical tools are used remove the polyp from the surrounding tissue. For fibroids, medications and hormone treatments can be used to temporarily improve the symptoms but do not treat the fibroids. To remove the fibroids, either a myomectomy (removal of fibroids while preserving the uterus) or hysterectomy (surgical removal of the uterus) can be performed.

One previous treatment of fibroids is uterine artery embolization (UAE). UAE introduces small particles into the uterine artery to block the arteries supplying blood to the fibroid. UAE is very painful and requires introducing the small particles into the circulatory system. Since the materials are being introduced into the uterine arteries, there is a risk that the particles may lodge in the wrong location or migrate to a region where treatment is not needed. Further, UAE is generally performed by a doctor other than the patient's primary gynecologist, which removes the patient from their primary care physician and can cause anxiety.

The present disclosure can provide solutions to these problems, and other problems, such as by providing methods and devices that can effectively treat the target tissue (e.g., uterine abnormality) with minimal equipment, by the gynecologist, without symptomatic treatment or hormone therapy, as well as not requiring the surgical removal of the uterus or the removal of the target issue from the patient. Additionally, the device and methods of the present disclosure can be performed as an out-patient procedure not requiring hospital admission.

The present disclosure provides devices and method that can treat target tissue without needing to surgically remove the target tissue. For example, a devascularizing material can be applied topically to the target tissue or injected directly into the target tissue. As discussed herein, the devascularizing material can treat the target tissue by reducing the biological activity of the target tissue. By reducing the biological activity, the blood supply within the target tissue is disrupted or abolished such that the target tissue can no longer survive and is thereby treated. Thus, the present disclosure provides a selective approach to treat a specific region (target tissue) while minimizing the risk of compromising collateral arterial flow.

This overview is intended to provide an overview of subject matter of the present patent application. It is not intended to provide an exclusive or exhaustive explanation of the invention. The detailed description is included to provide further information about the present patent application.

### BRIEF DESCRIPTION OF THE DRAWINGS

FIG. 1 illustrates an example of a uterus having various uterine abnormalities.
FIG. 2 is a schematic illustration of a surgical device having a treatment end to treat uterine abnormalities, according to one example of the present disclosure.
FIG. 3 is a schematic illustration of a surgical device having a treatment end to treat uterine abnormalities, according to one example of the present disclosure.
FIG. 4A is a close-up view of a treatment end of a surgical device treating a uterine fibroid according to one example of the present disclosure.
FIG. 4B is a cross-sectional view of the treatment end of the surgical device in FIG. 4A.
FIG. 4C is another cross-sectional view of the treatment end of the surgical device in FIG. 4A.
FIG. 5A is a close-up view of a treatment end of a surgical device treating a polyp, according to one example of the present disclosure.
FIG. 5B is a cross-sectional view of the treatment end of the surgical device in FIG. 5A.
FIG. 5C is another cross-sectional view of the treatment end of the surgical device in FIG. 5A.
FIG. 6 is a close-up view of a treatment end of a surgical device treating endometriosis, according to one example of the present disclosure.
FIG. 7 illustrates a flow chart of a method of treating target tissue with a devascularizating material.

In the drawings, which are not necessarily drawn to scale, like numerals may describe similar components in different views. Like numerals having different letter suffixes may represent different instances of similar components. The drawings illustrate generally, by way of example, but not by way of limitation, various examples discussed in the present document.

### DETAILED DESCRIPTION

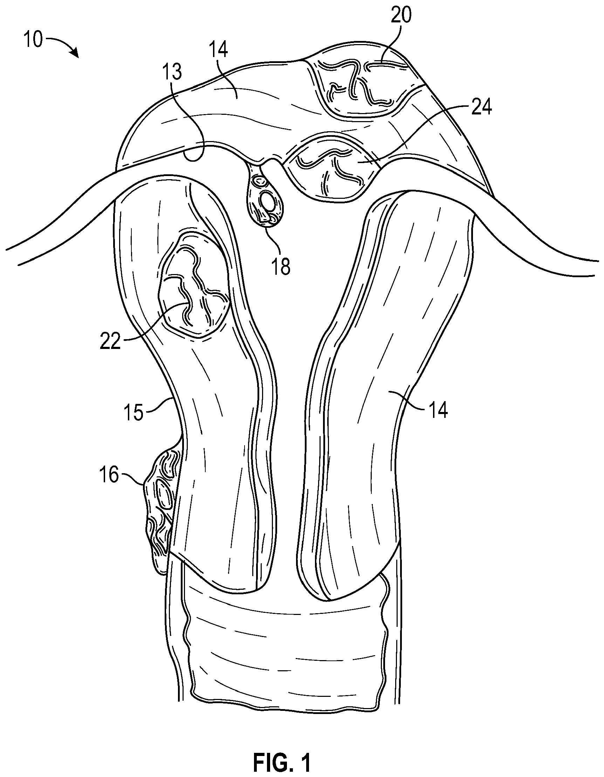

FIG. 1 illustrates different anatomical locations of various uterine abnormalities. Endometriosis 16 is endometrial tissue located on the external surface 15 of the wall 14 of the uterus 10. A polyp 18 is attached to the inner surface 13 of the wall 14. A sub-mucosal fibroid 24 is located on the inner surface 13 of the uterus 10. A sub-serosal fibroid 20 is located on the external surface 15 of the uterus 10. An intra-mural fibroid 22 is located within the wall 14 of the uterus 10.

The location, size and orientation of the uterine abnormalities is first determined by one or more known imaging techniques. For example, ultrasonic imaging (known as an "ultrasound") can be performed using a transducer placed externally of the patient's body or located within the uterus, for example, at the end of a transcervically inserted ultrasonic probe. Other known means for determining uterine abnormalities such as MRI could also be used.

Once the location, size and orientation of the (or each) uterine abnormality has been determined, the surgeon will determine how to access each of the abnormalities. For example, sub-mucosal fibroids 24 and polyps 18 are typically are accessed transcervically, whereas sub-serosal fibroids 20, intra-mural fibroids 22, and endometriosis 16 are typically transcutaneously accessed from the pelvic cavity (i.e., laproscopically accessed). However, the manner of accessing each target tissue also depends on the desired outcome of the procedure, the size of the target tissue, as well as the location of other target tissues within the uterus.

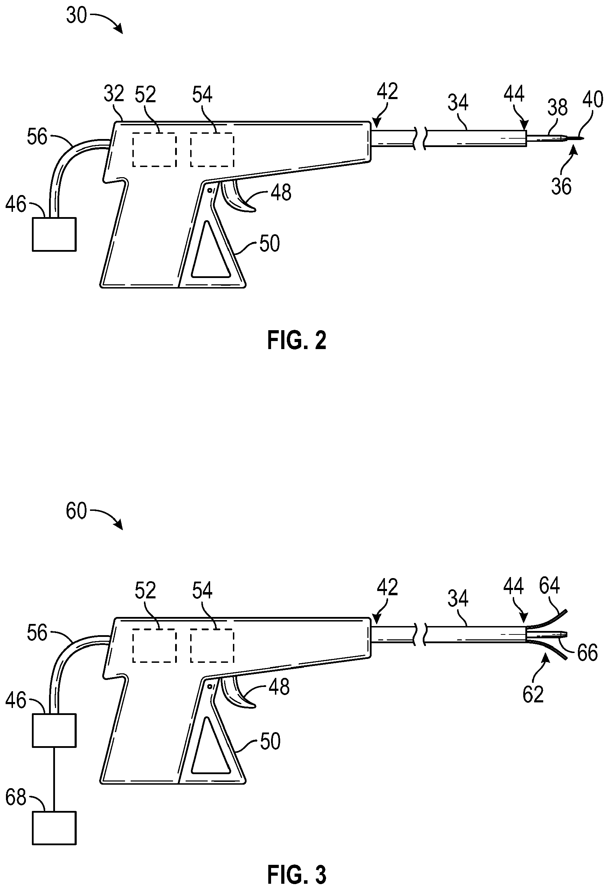

FIG. 2 is a schematic illustration of surgical device 30 according to the present disclosure. The surgical device 30 can administer the treatment material to the target tissue via injection. That is, the treatment material is injected directly into the target tissue.

Surgical device 30 can comprise handpiece or handle 32, outer shaft 34 and treatment end 36. Treatment end 36 can comprise an introducer shaft 38 and an injection needle 40. Outer shaft 34 can be a cannulated shaft extending from a proximal end portion 42 to a distal end portion 44. Surgical device 30 can further comprise a treatment material container 46 including the treatment material, e.g., a devascularizating material. The treatment material container 46 can be coupled to the handle 32 via a tubing 56 that is in fluid communication with the injection needle 40. While shown external to the handle 32, the treatment material container 46 can be positioned within the handle 32.

Handle 32 can comprise any device suitable for facilitating manipulation and operation of surgical device 30. Handle 32 can be located at the proximal end of proximal end portion 42 or another suitable location along outer shaft 34. In examples, handle 32 can comprise a pistol grip, a knob, a handlebar grip and the like. Handle 32 can further comprise one or more actuation devices 48, 50 that, when activated, actuate one or more mechanisms 52, 54 that perform one or more of the surgical device 30 features. Actuation devices 48, 50 can comprise one or more of buttons, triggers, levers, knobs, dials and the like.

Actuation devices 48, 50 can actuate mechanisms 52, 54 to, e.g., advance the introducer shaft 38 from the outer shaft 34, advance the injection needle 40 from the introducer shaft 38, and administer the treatment material to the target tissue.

Outer shaft 34 can comprise an elongate member configured to allow the treatment end 36 to be inserted into a patient. In examples, the outer shaft 34 can be sized for performing laparoscopic procedures in conjunction with a laparoscope. As such, the outer shaft 34 can be inserted into an incision in the epidermis of a patient, through a body cavity of the patient and into an organ. In examples, the shaft 34 can be inserted transcervically into the uterus. Thus, it is desirable for the diameter or cross-sectional shape of the outer shaft 34 to be as small as possible to facilitate minimally invasive surgical procedures. The outer shaft 34 can be rigid and formed from a metal or plastic material.

The surgical device 30 can also typically include additional passages through which other devices can be introduced. For example, an optical system and/or an imaging system (such as an ultrasonic transducer) can be provided near the distal end portion 44 of the outer shaft 34 or provided as separate devices that are introduced to the target tissue through passages of the surgical device 30. The optical system and/or imaging system can be used by the surgeon during the procedure to monitor the position of, and thereby precisely position, the introducer shaft 38 and the injection needle 40. Further, the optical system and/or imaging system can be used to immediately measure the treatment of the target tissue to determine if more treatment is needed or if the procedure is complete. One example of such an optical system is disclosed in US Provisional Patent Application 62/940,328, filed November 26, 2019, titled "Surgical devices with Integrated Lighting Systems," which is incorporated by reference in its entirety.

FIG. 3 is a schematic illustration of surgical device 60 that is similar to surgical device 30 except that surgical device 60 includes treatment end 62 instead of treatment end 36. Surgical device 60 has a treatment end 62 that can be used to topically administer the treatment material to the target tissue. That is, treatment end 62 can apply the treatment material directly to the surface of the target tissue.

Surgical device 60 can comprise handpiece or handle 32, outer shaft 34 and treatment end 62. Treatment end 62 can comprise an application shaft 66 and optionally include a containment device 64. That containment device 64 can be used to contain the administered treatment material to a desired area around the target tissue. The treatment material container 46 can be coupled to the handle 32 via the tubing 56 that is in fluid communication with the injection needle 40. Surgical device 60 can further comprise a pump such as a sprayer pump 68 that is configured to deliver the treatment material to the target tissue at a desired flow, pressure, and form (e.g., spray, drip, or other). Surgical device 30 can further comprise actuation devices 48, 50 that, when activated, actuate mechanism 52, 54 perform one or more of the surgical device 60 features. For example, mechanisms 52, 54 can advance the application shaft 38 from the outer shaft 34, advance the containment device 64 from the outer shaft 34, and administer the treatment material to the target tissue. Surgical device 60 can include the optical systems and/or imaging systems discussed above with respect to surgical device 30.

Surgical devices 30, 60 disclosed herein can apply the treatment material to the target tissue including such as, but not limited to, uterine abnormalities. As discussed herein, the treatment material can be a devascularizing material that can be applied topically or injected directly into the target tissue. As used herein, "devascularizing material" is defined to include any material that can reduce the biological activity of a target tissue. For example, devascularizing materials can either contract, seal, and clog/block blood capillaries/vessels such that the blood flow of the target site is reduced/blocked off and the tissue at the target site is treated, e.g., can no longer function and dies. In one example, the devascularizing material can reduce the biological activity by physically stopping the blood flow, stopping the biological movement (e.g., expansion/contraction) of the blood vessels, and/or cause an immune response such that the biological activity is stopped. As discussed herein, the methods to apply the devascularizing material directly to the target tissue either by direct injection or topical treatment allows the surgeon to selectively treat the target tissue while minimizing the risk of adverse treatment to other regions outside of the target tissue.

The devascularizing material can be selected from one or more biocompatible materials that are capable of reducing the biological activity to treat the target site, e.g., causing necrosis. Numerous devascluarlizing materials are contemplated. The type of devascularizing material and surgical device used can be based on the type of target tissue, disease state, and particular anatomy being treated.

The devascularizing material can be a liquid (fluid), a semi-solid, and a combination of a liquid and particulate (solid) material. The devascularizing material can be a non-adhesive agent or an adhesive agent. In some examples, the devascularizing material can be selected from, but not limited to, collagen, thrombin, particulates, medical adhesives (glues), sclerosing agents, copolymers, and polymers, among others. For example, the devascularizing material can include polymeric material, crosslinked polymeric material, a gel or gelatin, a hydrogel, a foam such as a gel foam, an emulsion, a lipid emulsion, comprises self-assembling material.

The devascularizing material, in some examples, comprises collagen, thrombin, lipiodol, gelatin, acrylic gelatin, tris-acryl gelatin, alginic acid, alginate, cellulose acetate, poly(vinyl acetate), poly(ethylene vinyl alcohol), ethylene vinyl alcohol copolymer (EVOH), a biodegradable poly(hydroxyl acid) or poly(vinyl alcohol) (PVA) or various mixtures thereof. In some examples, the devascularizing material comprises Marsembol. In some examples, the devascularizing material comprises an alkyl cyanoacrylate, such as n-butyl cyanoacrylate. In some examples, the devascularizing agent includes sclerosing agents such as sodium tetradecyl sulfate, polidocanol, and ethanolamine oleate. In some examples, the devascularizing material comprises a particulate material, including microspheres and/or non-spherical particles.

The composition and characteristics (e.g., viscosity, size of particulates) of the devascularizing material can be dependent on the type of target tissue, disease state and particular anatomy being treated. For example, if the devascularizing material is being applied to the surface of the target tissue, the viscosity of the devascularizing material can increase to minimize treating tissue besides the target tissue.

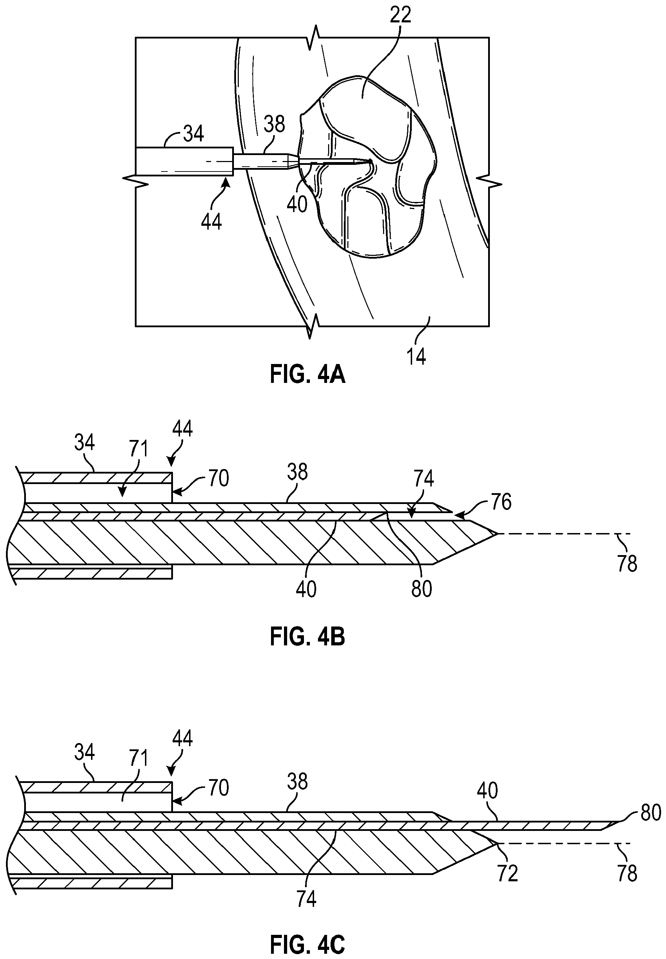

FIGS. 4A-C illustrate the treatment end 36 of the surgical device 30 shown in FIG. 2. FIG. 4A illustrates a close-up of the surgical device 30 treating an intra-mural fibroid 22, FIG. 4B illustrates a cross-sectional view of the treatment end 36 with the injection needle 40 positioned within the introducer shaft 38, and FIG. 4C illustrates a cross-sectional view of the treatment end 36 with the injection needle 40 extending from the introducer shaft 38.

The outer shaft 34 defines a lumen 71 extending along the length of the outer shaft 34 and an opening 70 at the distal end portion 44. The introducer shaft 38 can include a distal tip 72. The distal tip 72 can be positioned along a longitudinal axis 78 of the introducer shaft 38. The distal tip 72 can be sharp such that the introducer shaft 38 can penetrate tissue such as uterine tissue. The introducer shaft 38 can define a bore 74 extending along the body of the introducer shaft 38 to the opening 76. The opening 76 is adjacent to the distal tip 72. As shown in FIG. 4B and C, the bore 74 is offset from the longitudinal axis 78 of the introducer shaft 38.

The injection needle 40 can deliver the devascularizing material to the target tissue. The injection needle 40 can have a sharp-tip 80 such that the injection needle 40 can pierce tissue such as the fibroid 22 as the injection needle 40 is introduced/inserted into the fibroid 22 (target tissue). As seen in FIG. 4B, the injection needle 40 is positioned within the introducer shaft 38. During treatment, while positioned within the introducer shaft 38, the introducer shaft 38 can be advanced such that the distal tip 72 is positioned adjacent to the target tissue. In an example, the distal-tip 72 can pierce a portion of a uterine wall to access the target tissue, which in this example is a fibroid.

Once in a desired position, the injection needle 40 can be advanced from the introducer shaft 38. As seen in FIG. 4A, the introducer shaft 38 is positioned adjacent to the fibroid 22 and the injection needle 40 has been inserted into the fibroid. Once positioned within the target tissue, the user can activate the surgical device 30 to deliver the devascularizing material into the target tissue. One or more injections of the devascularizing material is contemplated and can depend on the type of devascularizing material used and the size of the target site.

While shown with the introducer shaft 38, it is contemplated that the devascularizing material can be delivered without the introducer shaft 38 such that the injection needle 40 is advanced from the outer shaft 34 and through any tissue surrounding the target tissue until the injection needle 40 is in a desired position within the target tissue.

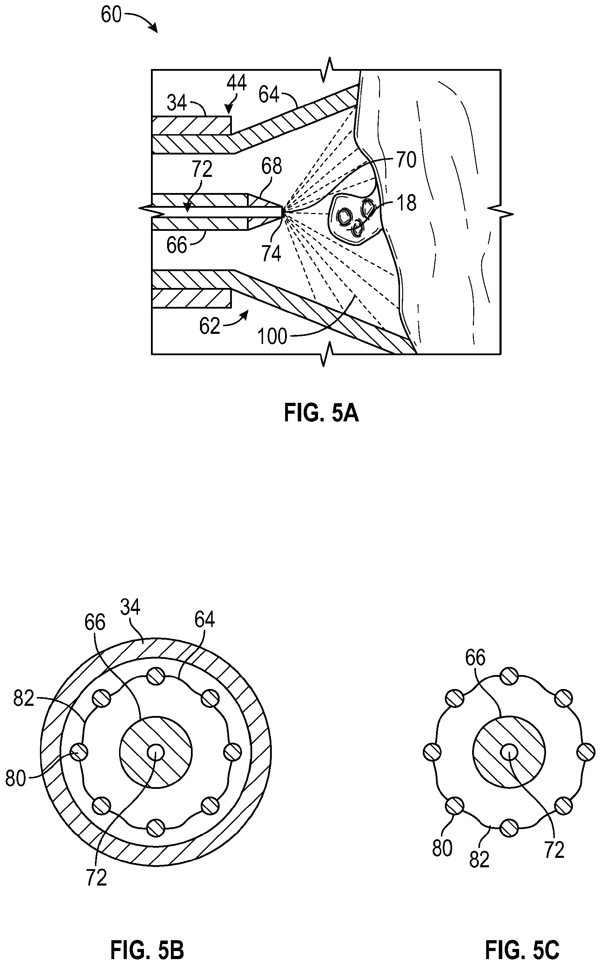

FIGS. 5A-C illustrate a close-up of a treatment end 62 of the surgical device 60 shown in FIG. 3. FIG. 5A illustrates the treatment end 62 treating a polyp 18 with the devascularizing material 100. FIG. 5B illustrates a cross-sectional view of the surgical device 60 when the containment device 64 is positioned within the outer shaft 34. FIG. 5C illustrates a cross-sectional view of the surgical device 60 when the containment device 64 is deployed.

The treatment end 62 can include the outer shaft 34 and an applicator such as an application shaft 66 including a nozzle 68. A bore 72 can extend along the length of the application shaft 66. The nozzle 68 includes a tip 74 defining an opening 70 and can be configured to apply the devascularizing material 100 to the target tissue in various ways. For example, depending on the anatomy, size of target tissue, and surrounding tissue, the nozzle 68 and/or pump 46 can vary the type of flow (e.g., stream, mist), area of coverage, and force that the devascularizing material 100 is applied to the target tissue.

The containment device 64 can have a collapsed position and a deployed position. The containment device 64 is in the collapsed position when the containment device 64 is positioned within the outer shaft 34. The containment device 64 is in the deployed position when the containment device 64 has been advanced from the outer shaft 34, as shown in FIG. 5A. The purpose of the containment device 64 is to minimize or prevent the devascularizing material 100 from migrating to areas that do not require treatment, e.g., during a surface treatment. In an example, the containment device 64 can include a plurality of flexible members 80 connected by a flexible material 82.

During treatment, the distal end portion 44 of the surgical device 60 can be advanced to a position adjacent to a target tissue. The containment device 64 can be advanced from the outer shaft 34 and positioned around the target tissue, e.g., polyp 18. Once the containment device 64 is in position, the application shaft 66 can advance out of the outer shaft 34 and to a predetermined distance from the target tissue. The distance can depend on the type of flow provided by the nozzle 68 and size of the target tissue. Once the application shaft 66 is in position the user can apply the devascularizing material to the target tissue for a predetermined time.

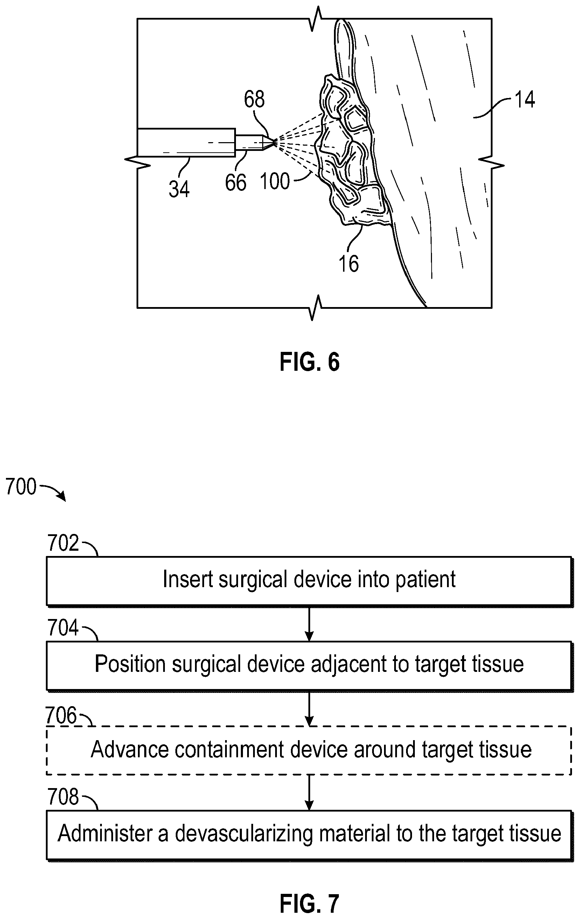

FIG. 6 illustrates a close-up of a treatment end 62 of the surgical device 60 shown in FIG. 3 but does not include the containment device 64. In certain instances, the location and type of target tissue can be treated topically without the containment device 64. During use, the outer shaft 34 can be advanced to a position adjacent to the target tissue such as endometriosis tissue 16. While shown without the containment device 64 (in FIG. 5A-C), endometriosis can be treated using the containment device. Once in position, the applicator shaft 66 can be advanced from the outer shaft 34 and positioned a predetermined distance from the target tissue. The predetermined distance can depend of a variety of variables such as a type of nozzle 68, devascularizing material 100, and size of target tissue. Once at the predetermined distance, the devascularizing material 100 can be applied to the target tissue.

In some examples, the treatment end 36 and treatment end 62 can be combined into a single device such that either treatment end 36 or treatment end 62 can be advanced from the outer shaft 34 to treat the target tissue. In this way, a patient having internal and external target tissues can be treated without having to remove the outer shaft. In one example, the treatment end 36 and the treatment end 62 can both fit within the outer shaft and be independently advanceable from the outer shaft 34. In one example, the treatment end 36 can be configured to be advanced within the containment device 64 such that while administering the devascularizing material to the surface of the target tissue, if the user determines an internal application of the devascularizing agent would be beneficial, the treatment end 36 can advance from the outer shaft 34 and the containment device 64 such that the containment device 64 does not have to be removed prior to administering the devascularizing agent to within the target tissue.

FIG. 7 illustrates a flow chart of method 700 of devascularizing a target tissue (uterine abnormality) with a surgical device. The method 700 can include inserting the surgical device into the patient (702), position a treatment end of the surgical device adjacent to target tissue (704), optionally, advance the containment device around the target tissue (706), and administer a devascularizing material to the target tissue (708).

In step 702, depending on the type and location of the uterine abnormality, the surgical device can be inserted transcervically or through a small incision in the patient. The surgical device can have an outer shaft with a treatment end translatable within the outer shaft and positioned at a distal end portion of the surgical device. In an example, the diameter of a cross-section of the distal end portion of the surgical device entering the patient is less than about 6 mm. This can, for example, allow for more efficient transcervical insertion with less patient pain.

Subsequently, in step 704, the operator can position the surgical device adjacent to the target tissue. For example, the operator can position the surgical device such that the distal end portion is adjacent to the target tissue. In an example where the devascularizing material is being injected directly into the target tissue, an injection needle can be inserted into the target tissue. As discussed herein, optionally, an introducer shaft can be used to pierce, e.g., the uterine wall and be positioned adjacent to (or slightly into the target tissue) and the injection needle can then advance from the introducer shaft and enter the target tissue to deliver the devascularizing agent.

In an example where the devascularizing material is being administered topically (i.e., to a surface of the target tissue), the operator can advance the application shaft out of the outer shaft such that the nozzle of the application shaft is positioned adjacent to the target tissue.

Optionally, at step 706, the containment device can be advanced from the outer shaft and expand. For example, the amount that the containment device advances out form the outer shaft can vary the area defined by the end of the containment device. Thus, size of the containment device can be varied based on the amount that the containment device is advanced from the outer shaft. In one example, the farther the containment device advances from the outer shaft the larger the distal end of the containment device. Once the containment device is deployed, the containment device can be positioned around the target tissue and in contact with tissue, e.g., uterine wall, surrounding the target tissue.

In step 708, the operator can administer a devascularizing material to the target tissue. As discussed herein, the devascularizing material can be applied either topically (to a surface) of the target tissue and/or it can be injected into the target tissue. Topical application of the devascularizing material can be performed using the surgical devices shown in FIGS. 3, 6, and 5A-C. Injecting the devascularizing material can be performed using the surgical devices shown in FIGS. 2 and 4A-C. Further, a combination of the topical treatment and the injection can be used to treat the target tissue.

The operator can determine the treatment outcome simultaneously during application or after application, such that the operator can determine if additional treatment is needed or if the treatment is complete. For example, visual means can be used to view the tissue and treatment outcome. Additionally, doppler technology can be used to view and determine the treated tissue from the untreated tissue and determine whether or not more treatment is needed to fully treat the target tissue. Once satisfied with the treatment, the operator can stop administering the devascularizing material and remove it from the patient.

The devices and methods disclosed herein provide a variety of benefits. For example, the devices and methods directly treat target tissue (such as uterine abnormalities). That is, the target tissue is treated with a devascularizing material either on a surface of the target tissue or is injected into the target tissue. This type of treatment can simplify the equipment needed, while fully treating the target tissue without requiring surgery or removal of tissue from the patient's body. The devices and methods disclosed herein provide a selective approach to treating target tissue by reducing the biological activity of the target tissue by applying the devascularizing material topically to the surface of the target tissue or injection the devascularizing material directly into the target tissue.

### Various Notes & Examples

Each of these non-limiting examples can stand on its own, or can be combined in various permutations or combinations with one or more of the other examples.

Example 1 provides a method for treating a target tissue, the method comprising: introducing a portion of a surgical device into a patient, the surgical device having a treatment end; positioning the treatment end of the surgical device adjacent to the target tissue; and administering a devascularizing material to the target tissue, wherein the devascularizing material is able to devascularize the target tissue.

In Example 2, the subject matter of Example 1 optionally includes where the administering the devascularizing material includes at least one of: applying the devascularizing material to a surface of the target tissue; and injecting the devascularizing material into the target tissue.

In Example 3, the subject matter of Examples 1-2 optionally includes where the devascularizing material includes a cyanoacrylate.

In Example 4, the subject matter of Examples 1-3 optionally includes where the surgical device includes: an outer shaft extending from a proximal end portion to a distal end portion; and a treatment end positioned at the distal end portion and configured to advance from the outer shaft to treat the target tissue.

In Example 5, the subject matter of Example 4 optionally includes where, when the administering the devascularizing material includes applying the devascularizing material directly to a surface of the target tissue, the treatment end includes: an application shaft having an elongated body and a nozzle positioned at a distal end of the elongated body.

In Example 6, the subject matter of Example 5 optionally includes where the treatment end further includes a containment device having a collapsed position, when positioned within the outer shaft, and a deployed position, when advanced from the outer shaft.

In Example 7, the subject matter of Example 6 optionally includes where, prior to administering the devascularizing material, the method further includes: advancing the containment device from the collapsed position to the deployed position; and positioning the containment device, in the deployed position, around the target tissue to be treated.

In Example 8, the subject matter of Example 4 optionally includes where, when the administering the devascularizing material includes injecting the devascularizing material directly into the target tissue, the treatment end includes: an injection needle positioned within the outer shaft, the injection needle configured to advance from the outer shaft and into the target tissue to administer the devascularizing material.

In Example 9, the subject matter of Example 8 optionally includes where administering the devascularizing material includes a plurality of injections into the target tissue, and wherein each injection of the plurality of injections includes administering an amount of the devascularizing material.

In Example 10, the subject matter of Example 4 optionally includes where, when the administering the devascularizing material includes injecting the devascularizing material directly into the target tissue, the treatment end includes: an introducer shaft extending from a proximal end to a distal tip, the introducer shaft defining a bore that is offset from the longitudinal axis; and an injection needle positioned within the bore of the introducer shaft and configured to advance from the introducer shaft and into the target tissue to administer the devascularizing material.

In Example 11, the subject matter of Examples 1-10 optionally includes where the target tissue is uterine tissue that includes a uterine abnormality.

In Example 12, the subject matter of Examples 11 optionally includes where the uterine abnormality includes one of a polyp, a fibroid, and endometriosis.

In Example 13, the subject matter of Examples 1-12 optionally includes where, prior to positioning the surgical device adjacent to the target tissue, the method includes distending a body cavity with a distension member.

In Example 14, the subject matter of Examples 1-3 optionally includes monitoring the target tissue to determine treated tissue from untreated tissue.

Example 15 provides a method for treating a target tissue, the method comprising: introducing a portion of a surgical device into a patient, the surgical device including: an outer shaft extending from a proximal end portion to a distal end portion defining a distal opening; and a treatment end positioned at the distal end portion of the outer shaft, the treatment end, including: an injection needle translatable within the outer shaft; positioning the treatment end of the surgical device adjacent to the target tissue; and advancing the injection needle from the opening and into the target tissue; and administering a devascularizing material within the target tissue via the injection needle, wherein the devascularizing material is able to devascularize the target tissue.

In Example 16, the subject matter of Example 15 optionally includes where the devascularizing material includes a cyanoacrylate.

Example 17 provides a method for treating a target tissue, the method comprising: introducing a portion of a surgical device into a patient, the surgical device including: an outer shaft extending from a proximal end portion to a distal end portion defining an opening; and a treatment end positioned at the distal end portion of the outer shaft, the treatment end, including: an introducer shaft translatable within the outer shaft and extending from a proximal end to a distal tip positioned along a longitudinal axis of the introducer shaft, the introducer shaft defining a bore and an opening, the opening adjacent to the distal tip; and an injection needle positioned within the bore of the introducer shaft; advancing the introducer shaft from the outer shaft to a position adjacent to the target tissue; advancing the injection needle from the opening of the introducer shaft and into the target tissue; and administering a devascularizing material within the target tissue via the injection needle, wherein the devascularizing material is able to devascularize the target tissue.

In Example 18, the subject matter of Example 17 optionally includes where the devascularizing material includes a cyanoacrylate.

Example 19 provides a method for treating a target tissue, the method comprising: introducing a portion of a surgical device into a patient, the surgical device including: an outer shaft extending from a proximal end portion to a distal end portion defining an opening; and a treatment end positioned at the distal end portion of the outer shaft, the treatment end, including: an application shaft having an elongated body and a nozzle positioned at a distal end of the elongated body; advancing the application shaft from the outer shaft to a position adjacent to the target tissue; and administering a devascularizing material to a surface of the target tissue via the nozzle.

In Example 20, the subject matter of Example 19 optionally includes where the devascularizing material includes a cyanoacrylate.

Example 21 provides a method for treating a target tissue, the method comprising: introducing a portion of a surgical device into a patient, the surgical device including: an outer shaft extending from a proximal end portion to a distal end portion defining an opening; and a treatment end positioned at the distal end portion of the outer shaft, the treatment end, including: an application shaft having an elongated body and a nozzle positioned at a distal end of the elongated body; and a containment device translatable within the outer shaft, the containment device having a collapsed position, when positioned within the outer shaft, and a deployed position, when advanced from the outer shaft; advancing the containment device from the collapsed position to the deployed position; positioning the containment device around the target tissue to be treated; advancing the application shaft from the outer shaft to a position adjacent to the target tissue and within the containment device; and administering a devascularizing material to a surface of the target tissue via the nozzle.

In Example 22, the subject matter of Example 21 optionally includes where the devascularizing material includes a cyanoacrylate.

Example 23 provides a surgical device for devascularizing target tissue, including: an outer shaft extending from a proximal end portion to a distal end portion defining an opening; and a treatment end positioned at the distal end portion of the outer shaft, the treatment end including: an application shaft having an elongated body and a nozzle positioned at a distal end of the elongated body; and a containment device translatable within the outer shaft, the containment device having a collapsed position, when positioned within the outer shaft, and a deployed position, when advanced from the outer shaft, the application shaft translatable within the outer shaft and configured to apply a devascularizing material onto a surface of the target tissue that is contained to the target issue via the containment device.

Example 24 provides a surgical device for devascularizing target tissue, including: an outer shaft extending from a proximal end portion to a distal end portion defining an opening; and a treatment end positioned at the distal end portion of the outer shaft, the treatment end including: an application shaft having an elongated body and a nozzle positioned at a distal end of the elongated body, the application shaft translatable within the outer shaft and configured to apply a devascularizing material onto a surface of the target tissue.

Example 25 provides a surgical device for devascularizing target tissue, including: an outer shaft extending from a proximal end portion to a distal end portion defining an opening; and a treatment end positioned at the distal end portion of the outer shaft, the treatment end including: an introducer shaft translatable within the outer shaft and extending from a proximal end to a distal tip along a longitudinal axis, the introducer shaft defining a bore and a distal opening that are offset from the longitudinal axis; and an injection needle positioned within the bore of the introducer shaft, the injection needle translatable within the introducer shaft and configured to inject a devascularizing material into the target tissue.

Example 26 provides a combination of any one of Examples 1 through 25.

The above detailed description includes references to the accompanying drawings, which form a part of the detailed description. The drawings show, by way of illustration, specific examples in which the invention can be practiced. These examples are also referred to herein as "examples." Such examples can include elements in addition to those shown or described. However, the present inventor also contemplates examples in which only those elements shown or described are provided. Moreover, the present inventor also contemplates examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their obj ects.

The above description is intended to be illustrative, and not restrictive. For example, the above-described examples (or one or more aspects thereof) may be used in combination with each other. Other examples can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed example. Thus, the following claims are hereby incorporated into the Detailed Description as examples or examples, with each claim standing on its own as a separate example, and it is contemplated that such examples can be combined with each other in various combinations or permutations. The scope of the invention should be determined with reference to the appended claims, along with the full scope of equivalents to which such claims are entitled.

## Claims

1. A surgical device for devascularizing target tissue, including:
an outer shaft extending from a proximal end portion to a distal end portion defining an opening;
a first treatment end configured to extend from the outer shaft and administer a devascularizing material to a surface of the target tissue; and
a second treatment end configured to extend from the outer shaft and administer the devascularizing material into the target tissue.

2. The surgical device of claim 1, wherein the first treatment end includes:
an application shaft having an elongated body and a nozzle positioned at a distal end of the elongated body; and
a containment device having a collapsed position, when positioned within the outer shaft, and a deployed position, when advanced from the outer shaft.

3. The surgical device of any one of claims 1 and 2, wherein the second treatment end includes:
an introducer shaft extending from a proximal end to a distal tip, the introducer shaft defining a bore that is offset from the longitudinal axis; and
an injection needle positioned within the bore of the introducer shaft.

4. The surgical device of any one of claims 1 through 3, wherein surgical device further includes:
a handle, wherein the outer shaft extends from the handle, the handle including:
one or more actuators configured to:
advance the first treatment end from the outer shaft;
advance the second treatment end from the outer shaft; and
administer the devascularizing material to the target tissue.

5. A surgical system for devascularizing target tissue, including:
a surgical device, including:
a handle; and
an outer shaft extending from a proximal end portion to a distal end portion defining an opening;
a first treatment end configured to extend from the outer shaft and administer a devascularizing material to a surface of the target tissue; and
a second treatment end configured to extend from the outer shaft and administer the devascularizing material into the target tissue;
a treatment material container in fluid communication with the first treatment end and the second treatment end, the treatment material container including the devascularizing material and
a pump configured to administer the devascularizing material from the treatment material container to at least one of the first treatment end and the second treatment end.

6. The surgical system of claim 5, wherein the first treatment end includes:
an application shaft having an elongated body and a nozzle positioned at a distal end of the elongated body; and
a containment device having a collapsed position, when positioned within the outer shaft, and a deployed position, when advanced from the outer shaft.

7. The surgical system of any one of claims 5 and 6, wherein the second treatment end includes:
an introducer shaft extending from a proximal end to a distal tip, the introducer shaft defining a bore that is offset from the longitudinal axis; and
an injection needle positioned within the bore of the introducer shaft.

8. The surgical system of any one of claims 5 through 7, wherein the devascularization material includes a cyanoacrylate.

9. The surgical system of any one of claims 5 through 8, wherein surgical system further includes one or more actuators configured to:
advance the first treatment end from the outer shaft;
advance the second treatment end from the outer shaft; and
administer the devascularizing material to the target tissue.

10. The surgical system of any one of claims 5 through 9, further including:
an imaging system configured to monitor the position of at least one of the first treatment and the second treatment end.

11. A method for treating a target tissue, the method comprising:
introducing a portion of a surgical device into a patient, the surgical device having a treatment end;
positioning the treatment end of the surgical device adjacent to the target tissue; and
administering a devascularizing material to the target tissue, wherein the devascularizing material is able to devascularize the target tissue.

12. The method of claim 11, wherein the administering the devascularizing material includes at least one of:
applying the devascularizing material to a surface of the target tissue; and
injecting the devascularizing material into the target tissue.

13. The method of any one of claims 11 and 12, wherein the devascularizating material includes a cyanoacrylate.

14. The method of claim 11, wherein the treatment end further includes an application shaft having an elongated body and a nozzle positioned at a distal end of the elongated body, wherein the administering the devascularizing material includes delivering the devascularizing material through the nozzle to the target tissue.

15. The method of claim 11, wherein the treatment end further includes:
an introducer shaft extending from a proximal end to a distal tip, the introducer shaft defining a bore that is offset from the longitudinal axis; and
an injection needle positioned within the bore of the introducer shaft, wherein administering the devascularizing material includes:
advancing the introducer shaft from the outer shaft to a position adjacent to the target tissue;
advancing the injection needle from the opening of the introducer shaft and into the target tissue; and
administering the devascularizing material within the target tissue via the injection needle.
